# EUROPÄISCHE PATENTSCHRIFT

(11) **EP 0 332 864 B1**
(45) Veröffentlichungstag und Bekanntmachung des Hinweises auf die Patenterteilung: **27.01.1993**
(21) Anmeldenummer: 89102612.2
(22) Anmeldetag: 16.02.1989
(51) Int. Cl.: C12M 3/00, C12N 15/89

(54) **Verfahren zur Mikroinjektion in lebende Zellen und Vorrichtung zur Durchführung des Verfahrens**
Process for microinjection into living cells and apparatus therefor
Procédé de micro-injection dans des cellules vivantes et appareillage pour la mise en oeuvre du procédé

(30) Priorität: 15.03.1988 DE 3808531
(43) Veröffentlichungstag der Anmeldung: 20.09.1989
(73) Patentinhaber: EPPENDORF-NETHELER-HINZ GMBH, D-22339 Hamburg (DE)
(72) Erfinder: Bertholdt, Günther, Dipl.Bio.Dr., D-7406 Mössingen 5 (DE)
(74) Vertreter: Hauck, Hans, Dipl.-Ing.

(56) Entgegenhaltungen:
- GB-A- 2 022 287
- REVUE GENERALE DE L'ELCTRICITE, Nr. 11, 1984, Paris (FR); C.DIAZ et al., Seiten 747-751*

## Beschreibung

Die Erfindung betrifft ein Verfahren zur Mikroinjektion von Flüssigkeiten oder Suspensionen von Stoffen in lebende Zellen, mit einer bewegbar gelagerten Kanüle und einem Tisch, wobei die schräg zu diesem eingestellte Kanüle und der Tisch verbunden sind und diese Teile relativ zueinander in einer Ebene parallel zum Tisch in zwei Richtungen (x-y-Richtungen) einstellbar sind und ferner die Kanüle in einer zu der durch die x-y-Richtung bestimmten Ebene senkrechten z-Richtung bewegbar ist.

Ferner bezieht sich die Erfindung auf eine Vorrichtung zur Durchführung des Verfahrens mit einem Halter für die Kanüle.

Der Ausdruck Kanüle erfaßt auch Kapillaren bzw. Mikrokapillaren.

Dabei ist bereits eine gewisse Mechanisierung angestrebt worden, bei welcher insbesondere unter gleichzeitiger Beobachtung durch ein Mikroskop eine schräg zur Senkrechten bzw. dem Tisch eingestellte Kanüle nach ihrer Ausrichtung über einer Zelle senkrecht nach unten bewegt wird. Probleme ergeben sich dabei bei einer Abschätzung der Position einer Kanüle bzw. Kapillare in der z-Richtung. Hierbei ist auch zu berücksichtigen, daß bei der Mikroinjektionstechnik nicht immer beabsichtigt ist, den Zellkern einer Zelle als Injektionsort vorzusehen, auch wenn dieser den Injektionsort bilden kann. Die bekannten Ausführungen lassen lediglich eine grobe Abschätzung des gewünschten Einstichortes zu. Die Bewegung senkrecht nach unten hat den Nachteil, daß dabei die Kanüle mit einer erheblichen Seitenkomponente in die Zelle eingedrückt wird und dabei die Zellenmembran in länglicher Form aufreißt. Dadurch können die Zellenmenbranen und damit die Zelle irreversibel geschädigt werden. Außerdem besteht die Gefahr, daß eine Kanüle abbrechen kann.

Es ist bekannt, als Tisch die Platte eines Mikroskopes vorzusehen, wobei ein Halter der Kanüle so angeordnet ist, daß die Spitze der Kanüle in der Beobachtungsrichtung liegt und eine genaue Beobachtung dadurch gesichert wird, daß die Spitze durch Fokussieren des Mikroskopes genau erfaßt wird.

Dieser zu einem Mikroskop gehörende Tisch ist dabei in bekannter Weise ein sogenannter Kreuztisch, welcher zum Objektiv einstellbar ist. Das hat den Zweck, jeweils eine oder mehrere von zahlreichen Zellen einer Zellkultur auf dem Kreuztisch in den Beobachtungsbereich des Mikroskopes zu bringen.

Unter diesem Gesichtspunkt geht die Erfindung in einer bevorzugten Ausführungsform davon aus, daß zahlreiche Zellen in einer bestimmten Anordnung, z.B. ein spezielles Zellkulturgefäß, auf dem Kreuztisch angeordnet sind und dann jeweils einzeln positionierbar sind.

Dafür sind Vorrichtungen bekannt, die mit erheblichem Aufwand arbeiten und mit Computereinsatz einen automatisch steuerbaren Kreuztisch antreiben, um jeweils eine Zelle nach Maßgabe eines geplanten Anordnungsmusters zu der Kanüle auszurichten.

Es sind weiterhin aufwendige Steuerungen der Kanüle durch computerisierte Bildverarbeitungstechniken denkbar, die dann zusätzlich relativ aufwendige Steuerungen des Mikroskoptisches zur Kanülenspitze erforderlich machen.

Der Erfindung liegt die Aufgabe zugrunde, ein Verfahren und eine Vorrichtung der eingangs angegebenen Art dahingehend zu verbessern, daß bei einer Erleichterung einer genauen Einstellung einer Kanüle zu jeweils einer Zelle ein axialer Einstich der Kanüle in die Zelle möglich ist.

Diese Aufgabe wird durch das Verfahren gemäß kennzeichnendem Teil des Anspruches 1 gelöst.

Die Verwendung der Kanüle als Zeiger zur direkten Markierung der beabsichtigten Einstichstelle und die dann erfolgende Rückwärtsbewegung ermöglichen eine genaue Durchführung der Mikroinjektion ohne Überlastung der Person, wobei zusätzlich trotz der visuell zu überwachenden Einstellung der Kanüle bei deren schräger Anordnung ein axialer Einstich möglich ist.

Dabei wird durch die Rückwärtsbewegung vor der Einstichbewegung nach der manuell durchgeführten Ausrichtung eine von der Konfiguration der Anordnung abhängige Automatik ausgelöst, um die Kanüle zunächst aus ihrer Ausrichtung zur Zelle herauszubewegen, um sie dann aus der gleichen Ebene von einer zurückversetzten Position aus axial einstechen zu können. Dieses Verfahren läßt sich besonders günstig durchführen, wenn parallel zum Tisch Ebenen gewählt werden, welche einerseits für die Bewegung der Kanüle und andererseits als Bemessungsgröße für die Einstichbewegung der Kanüle vorgesehen sind. Dabei ergeben sich besondere Vorteile aus den Ansprüchen 2 und 3.

Die oben genannte Aufgabe wird durch die Vorrichtung nach Anspruch 4 gelöst. Dabei ist zu berücksichtigen, daß eine solche Vorrichtung in verschiedener Weise ausgeführt sein kann. Sofern die Vorrichtung mit ihrem Betätigungsgerät in Verbindung mit einem oder zu einem Tisch vorgesehen ist, der als Kreuztisch in der einen Ebene in x-y-Richtung bewegbar ist, dann genügt es, im Betätigungsgerät nur eine erste Antriebsvorrichtung in der angegebenen Weise und eine zweite Antriebsvorrichtung in der z-Richtung anzuordnen. Die Einstellung der Kanüle zu einer Zelle könnte dann auch mit Hilfe der Bewegung des Kreuztisches vorgenommen werden, wobei dann im wesentlichen der Kreuztisch mit der Zelle verlagert wird.

Bevorzugt wird aber, daß das Betätigungsgerät zwei erste Antriebsvorrichtungen in der einen Ebene in x-y-Richtung hat. Entsprechend den Antriebsvorrichtungen sind die Führungseinrichtungen angeordnet, die als hinterschnittene Bahnen und/oder Schienen ausgeführt sein können, wie es beispielsweise an Werkzeugmaschinen bekannt ist.

Schon das Betätigungsgerät für sich ist eine besondere Ausführungsform zur Erfüllung der Aufgabe. In einer vorteilhaften Ausgestaltung ist dabei das Betätigungsgerät mit einer Festlegungsanordnung, beispielsweise in Form einer Klemmeinrichtung, zur starren Verbindung mit einem Tisch ausgeführt. Dieses ermöglicht auch einen nachträglichen Anbau an einem Tisch, besonders auch an einem Mikroskoptisch oder dem Tisch anderer Beobachtungseinrichtungen. Daraus ergibt sich, daß die erwähnten Antriebsvorrichtungen nicht mit den Antriebsvorrichtungen eines Kreuztisches eines Mikroskopes identisch sind. Sie haben aber vorteilhaft gleiche Antriebsvorrichtungen in den x-y-Richtungen, d.h. der Ebene des Kreuztisches. Dadurch läßt sich die als Zeiger verwendete Kanülenspitze beliebig über einer Zelle einstellen, welche in die Beobachtungsstrecke des Mikroskopes unter dem Kondensor gebracht worden ist.

In einer zweckmäßigen Ausgestaltung ist das Betätigungsgerät einteilig mit einem Tisch ausgeführt.

Weitere vorteilhafte Merkmale der Erfindung gehen aus den weiteren Ansprüchen hervor.

Die Erfindung wird im folgenden anhand von Ausführungsbeispielen erläutert, die in der Zeichnung dargestellt sind. In der Zeichnung zeigen:
Fig. 1 eine schematische Seitenansicht einer erfindungsgemäßen Vorrichtung;
Fig. 2 eine schematische Draufsicht auf eine erfindungsgemäße Vorrichtung gemäß Fig. 1 in Teildarstellung, jedoch mit einer Erweiterung;
Fig. 3 a) - c) drei schematische Darstellungen in Seitenansicht und stark vergrößertem Maßstab zur Erläuterung der Kanülen-Einstellung;
Fig. 4 eine perspektivische Ansicht einer Vorrichtung in schematischer Teildarstellung;
Fig. 5 ein schematisches Prinzipschaltbild für das Betätigungsgerät.

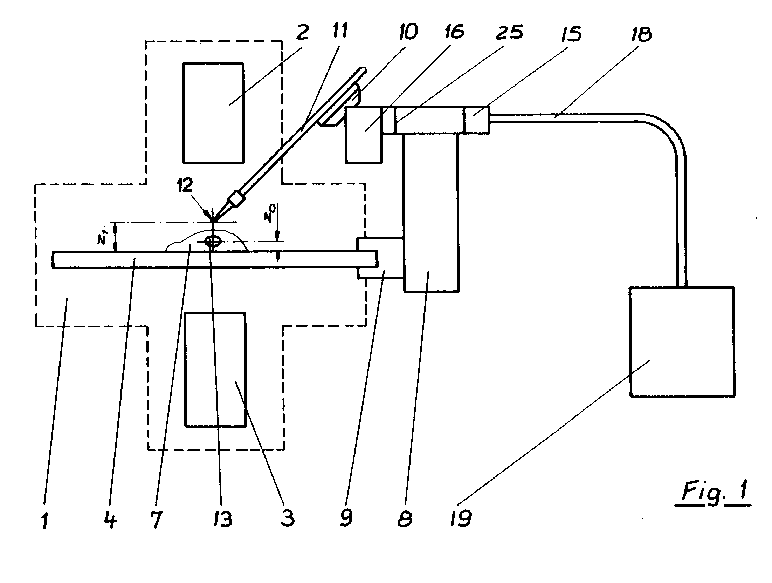

Gemäß Fig. 1 ist durch die gestrichelte Linie ein Mikroskop 1 schematisch angegeben, das einen Kondensor 2 und ein Objektiv 3 hat. Hierbei handelt es sich um ein inverses Mikroskop.

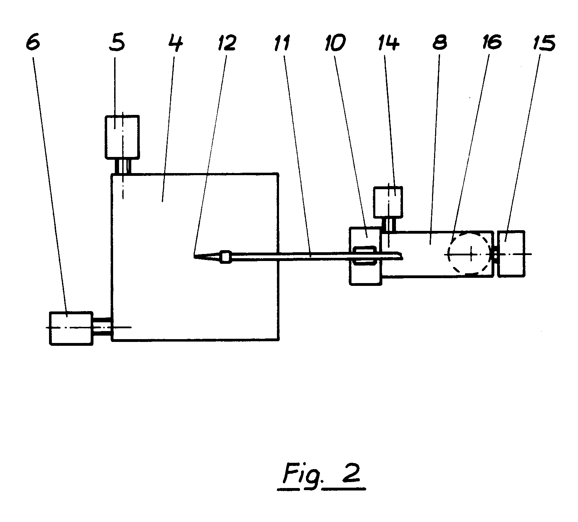

In diesem Mikroskop ist ein Kreuztisch 4 in bekannter Weise gelagert und mittels zweier Antriebsvorrichtungen 5, 6 (Fig. 2) in bezug zur Beobachtungslinie zwischen 2 und 3 einstellbar. Dieses hat im vorliegenden Fall den Zweck, eine Zelle 7, die auf dem Kreuztisch 4 neben zahlreichen Zellen angeordnet ist, in die Beobachtungslinie zwischen Kondensor 2 und Objektiv 3 einzustellen.

An dem Kreuztisch ist ein Betätigungsgerät 8 angeordnet, das entweder einteilig mit dem Kreuztisch ausgeführt ist oder aber durch eine Befestigungsanordnung 9 starr mit dem Kreuztisch 4 verbindbar ist.

An dem Betätigungsgerät ist bewegbar ein Halter 10 mit einer Kanüle 11 angeordnet. Diese Kanüle ist in einem Winkel, vorteilhaft von 45°, zum Kreuztisch angeordnet. Diese Kanüle 11 hat eine Spitze 12. Diese Spitze dient als Zeiger und ist durch Antriebsvorrichtungen so einstellbar, daß sie über den Kern 13 der eingestellten Zelle 7 gelangt.

Diese Antriebsvorrichtungen zur Ausrichtung des von der Spitze gebildeten Zeigers zur Zelle können diejenigen des Kreuztisches, aber auch die für den Halter 10 am Betätigungsgerät 8 sein. Beide Antriebe können auch kombiniert werden.

In einer grundlegenden Ausführung ist die als Zeiger vorgesehene Spitze der Kanüle in einer Ausgangsstellung angeordnet und der Kreuztisch 4 wird unter Beobachtung durch das Mikroskop so eingestellt, daß der Zeiger über einem Zellkern 13 angeordnet ist.

Die sich anschließenden Bewegungen der Kanüle werden durch die Antriebsvorrichtungen an dieser durchgeführt.

Fig. 1 zeigt dabei eine zweite Antriebsvorrichtung 16 zur Höheneinstellung und eine Antriebsvorrichtung 15 zu einer Bewegung parallel zum Tisch 4 in der Richtung der Kanüle 11.

In Fig. 2 sind schematisch das Betätigungsgerät 8, der Halter 10 und die Kanüle 11 gezeigt. In der Ebene des Kreuztisches 4, d.h. in der sogenannten x-y-Ebene, sind zwei Antriebsvorrichtungen 14, 15 senkrecht zueinander, jedoch in einer Ebene vorgesehen, die zur Ebene des Tisches 4 parallel ist. Sowohl die Antriebsvorrichtungen 5, 6 als auch die Antriebsvorrichtungen 14, 15 können zur Einstellung der Spitze 12 der Kanüle 11 zu einer Zelle auf dem Kreuztisch eingesetzt werden. Wenn dazu nur die Antriebsvorrichtungen 5, 6 eingesetzt werden, genügt in der dargestellten Ausführung, daß als erste Antriebsvorrichtung des Betätigungsgerätes 8 nur die Antriebsvorrichtung 15 zur Bewegung in Richtung der Kanüle 11 und die zweite Antriebsvorrichtung 16 angeordnet ist, die in der sogenannten z-Richtung, d.h. senkrecht zu der durch die Antriebe 14, 15 in der x-y-Richtung bestimmten Ebene vorgesehen ist.

Die Erweiterung in Fig. 2 gegenüber Fig. 1 ist die Hereinnahme der Antriebsvorrichtung 14. Es versteht sich, daß dann noch zwischen dem einen Querschnitt mit der Antriebsvorrichtung 14 und dem in der Höhe einstellbaren Geräteteil ein Zwischenschlitten angeordnet ist, der an verschiedenen Seiten senkrecht zueinander verlaufende Führungsanordnungen hat.

In Fig. 1 sind spezielle Führungsanordnungen für den Halter 10 in bezug zum Betätigungsgerät 8 nicht gezeigt. Sie können in üblicher Weise in Form hinterschnittener Führungsbahnen, Schienen oder dergleichen ausgeführt sein.

Ferner ist der Halter 10 durch eine nur schematisch angedeutete Einstellanordnung 25 zur Einstellung des Winkels der Kanüle 11 zum Tisch 4 vorgesehen.

Bezüglich Fig. 1 befindet sich die Spitze 12 der Kanüle 11 in einer Höhe z₁ über dem Kreuztisch 4. Diese Ebene z₁ liegt oberhalb der Zelle 7. Eine weitere Größe ist eine Ebene z₀, die in Fig. 1 dargestellt ist und auch über dem Kreuztisch 4 liegt, aber den Zellkern 13 schneidet. Daraus ist ersichtlich, daß die Spitze 12 der Kanüle 11 in einer Ebene in Höhe z₁ bewegbar ist, um über den Zellkern 13 entsprechend einer Beobachtung eingestellt zu werden.

In Fig. 1 sind rein formal eine Zuführungsleitung 18 und ein Zuführungsgerät 19 für die Zuführung der Substanz zur Kanüle eingezeichnet, wobei 18 eine Funktionsleitung ist, durch welche zugleich Steuerimpulse übertragen werden.

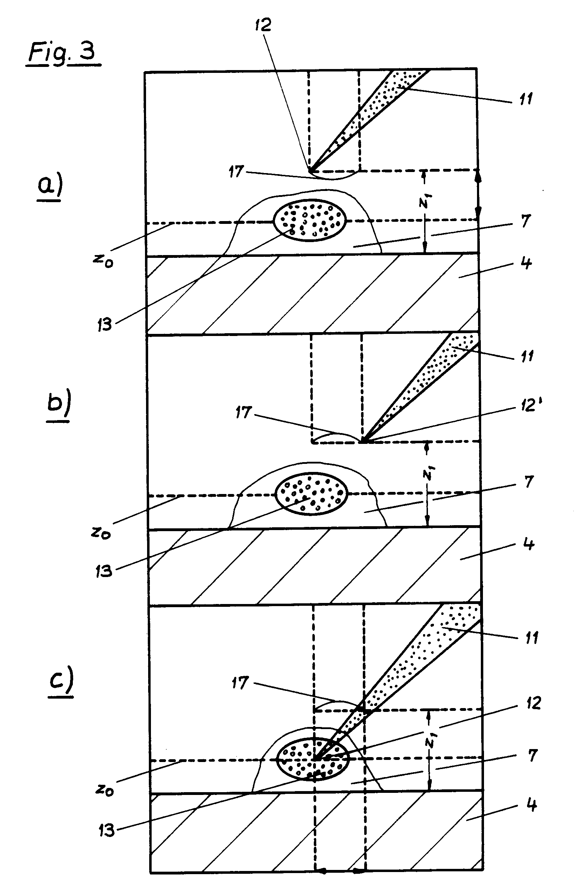

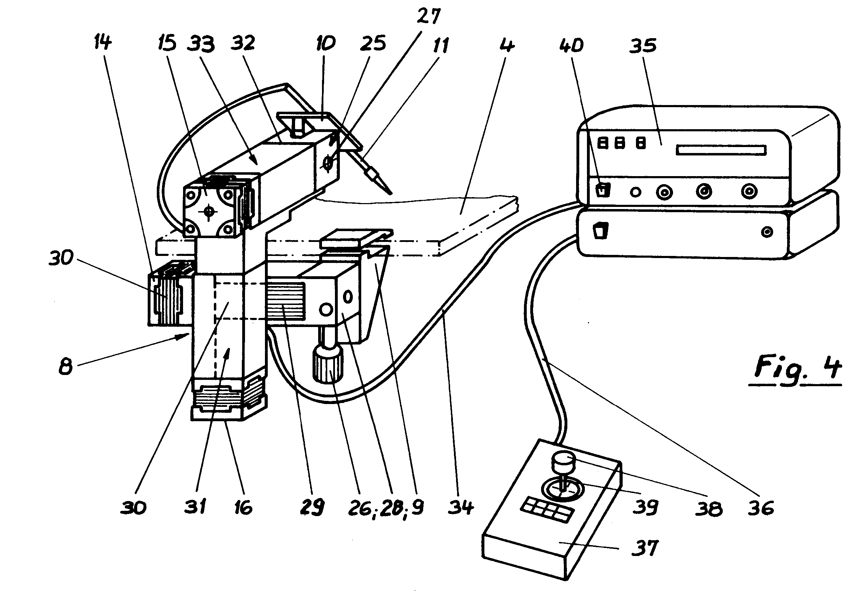

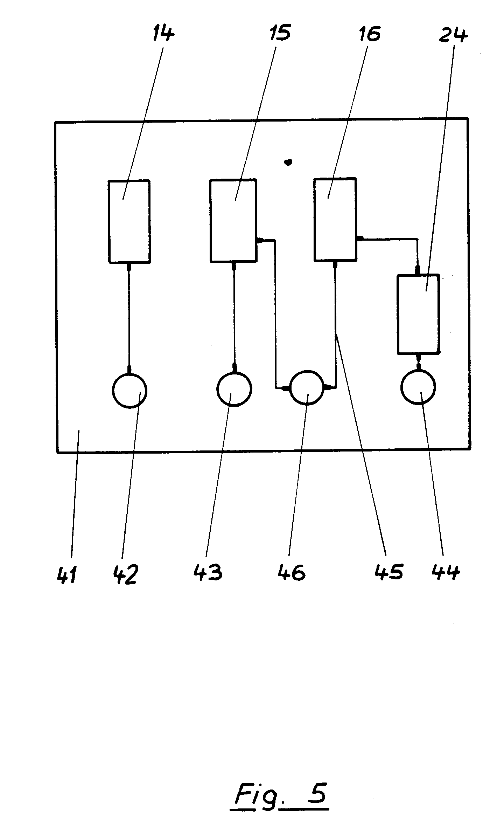

Für die Durchführung des Verfahrens und den Betrieb der Vorrichtung wird entweder durch Beobachtung oder aus Erfahrung eine Ebene parallel zum Kreuztisch 4, d.h. zur Auflagefläche des Kreuztisches über dieser in einer solchen Höhe eingestellt, daß sie den Zellkern 13 schneidet. Diese Ebene hat den Zweck, bei einer Bewegung der Kanüle 11 diese Bewegung dann anzuhalten und eine Rückbewegung auszulösen, wenn die Spitze 12 der Kanüle 11 die Mitte des Zellkerns 13 erreicht hat. Dieses wird insbesondere anhand der Fig. 3 noch verdeutlicht.

In den Fig. 3 a) bis c) ist jeweils ein Ausschnitt des Kreuztisches 4 mit der Zelle 7 und dem Zellkern 13 gezeigt. Die Kanüle 11 ist mit einer Spitze 12 über dem Zellkern angeordnet, und zwar in der Ebene, die um die Strecke z₁ über der Auflagefläche des Kreuztisches 4 liegt. Diese Ebene wird daher auch als z₁-Ebene bezeichnet.

Erkennbar ist in Figuren 3 a) bis c) auch eine Ebene z₀, welche den Kern 13 der Zelle schneidet.

Die Spitze 12 der Kanule 11 wird gemäß Fig. 3a) entsprechend einer Kontrolle nach Fig. 1 über den Zellkern 13 eingestellt. Nach dieser Einstellung wird der Halter 10 bezüglich Fig. 1 bzw. die Kanüle 11 parallel zur Ebene des Kreuztisches 4 um eine Strecke 17 in einer Richtung, beispielsweise der x-Richtung bewegt, so daß dann die Spitze 12′ so eingestellt ist, daß sie bei einer axialen Bewegung durch gleichzeitigen Antrieb, beispielsweise der Antriebsvorrichtung 15 und 16, axial in den Zellkern 13 einsticht (Fig. 3 c). Diese Einstichbewegung wird dadurch begrenzt, daß gemäß obigen Darlegungen die axialen Vorschubantriebe abgeschaltet werden, wenn die Spitze 12 die Ebene z₀ erreicht. Gleichzeitig besteht dann die Möglichkeit der Umsteuerung und Rückwärtsbewegung, so daß dann die Spitze 12 als Zeiger in einer Ebene z₁ auch durch Verstellung des Kreuztisches 4 und dann der Antriebsvorrichtungen 14, 15 wieder in die zum Kreuztisch 4 vertikale Projektion eines anderen Zellkerns 13 gebracht werden kann.

Besonders zeigt die Fig. 3 eine Kanüle mit einer 45° Winkeleinstellung. In dieser Ausführung ist die Höhendifferenz zwischen den Ebenen z₁ und z₀ gleich der Strecke 17, wie besonders deutlich aus Fig. 3 c) hervorgeht. Der gleichzeitige Antrieb in senkrechter und horizontaler Richtung bewegt daher die Kanüle 11 in der 45° Richtung zum Tisch 4 und zum axialen Einstich in die Zelle 13.

In Fig. 4 ist der Tisch 4, beispielsweise eines Mikroskopes, gezeigt. An diesem Tisch ist das Betätigungsgerät 8 angeordnet, und zwar mittels einer Befestigungsvorrichtung 9, die beispielsweise durch einen Stellknopf 26 anspannbar ist.

An dem Betätigungsgerät sind die drei Antriebsvorrichtungen 14, 15 und 16 für die beschriebenen Bewegungen vorgesehen, wobei die Fig. 4 eine gegenständliche Ausführung zeigt. Der Halter 10 mit der Kanüle 11 ist durch die Einstellanordnung 25 winkelmäßig einstellbar, wozu ein Betätigungsknopf 27 dient.

An dem Sockel des Betätigungsgerätes ist eine Führungsbaugruppe 28 befestigt, die parallel zum Tisch verlaufende Führungsschienen 29 für einen nicht erkennbaren und gestrichelt angedeuteten Schlitten 30 hat, der damit parallel zur Führungsbaugruppe seitwärts verlagerbar ist.

In diesem Schlitten 30 sind andererseits senkrecht gerichtete Führungsschienen-Anordnungen für eine Führungsbaugruppe 31 vorgesehen, die die Antriebsvorrichtung 16 hat, die in z-Richtung antreibt.

An dieser Führungsbaugruppe ist ein rechtwinklig angesetzter Ausleger 32 mit einer nicht dargestellten schienenartigen Führung für eine dritte Führungsbaugruppe 33 vorgesehen, die parallel zur Ebene des Tisches, aber senkrecht zur Bewegungsrichtung an der Führungsbaugruppe 28 bewegbar ist. Sie wird daher entsprechend Fig. 2 durch die Antriebsvorrichtung 15 angetrieben.

Dieses zeigt eine einfache Ausführung des Betätigungsgerätes 8. Dieses ist im gezeichneten Ausführungsbeispiel durch die Steuerleitung 34 an Steuereinheiten 35, beispielsweise eines Mikroskopes, bzw. zur Bewegung des Kreuztisches 4 angeschlossen. Über eine Funktionsverbindung 36 ist ein Steuergerät 37 mit einem Handhebel 38 angeschlossen, dessen Auslenkung in den verschiedenen Richtungen eine Bewegung in der x-y-Richtung auslöst. Eine Bewegung in der z-Richtung kann durch Drehung oder Einrückung eines Elementes am Steuergriff 39 ausgelöst werden.

Ob nun die Antriebsvorrichtungen des Kreuztisches 4 oder des Betätigungsgerätes 8 eingesetzt werden, kann durch Einstellung eines Hebels 40 bestimmt werden.

Ein schematisches Schaltbild nach Fig. 5 besitzt für das Betätigungsgerät 8 eine Steuerbaugruppe 41. In dieser sind die drei Antriebsvorrichtungen 14, 15 und 16 untergebracht bzw. bei der räumlich verteilten Anordnung nach Fig. 4 die entsprechend benannten Schalteinrichtungen. Dabei ist erkennbar, daß für die Antriebsvorrichtungen 14, 15 in der x-y-Richtung Betätigungshebel 42, 43 vorgesehen sind, die eine getrennte Einschaltung ermöglichen. Die Antriebsvorrichtung 16 für eine Bewegung in der z-Richtung ist mit einer Meßeinrichtung 24 verbunden. Diese hat einen Betätigungshebel 44, welcher eine Einstellung des Abstandes der z₀- und z₁-Ebene zuläßt. Daher ist die Antriebsvorrichtung 16 nach Maßgabe dieser Einstellung bewegbar.

In der soweit dargestellten Ausführungsform ist zwischen den Antriebsvorrichtungen 15, 16 eine Steuerschleife 45 mit einer Betätigungsvorrichtung 46 angeordnet. Deren Betätigung öffnet alle anderen Energieanschlüsse zu den Antriebsvorrichtungen 15 bis 16 und setzt nur die Antriebsvorrichtungen 15 und 16 mit gleicher Geschwindigkeit ein, wenn die Kanüle gemäß obigen Darlegungen in einem Winkel von 45° angeordnet ist. Dazu wird bemerkt, daß insbesondere die Antriebsvorrichtungen 15, 16 umsteuerbar sind, weil auch unter Einfluß der Meßeinrichtung 24 dann, wenn die Spitze 12 der Kanüle 11 die Ebene z₀ erreicht, automatisch eine Umsteuerung und Rückbewegung der Kanülenspitze 12 in die Ebene z₁ erfolgt.

Zu der gegenständlichen Ausführung des Betätigungsgerätes 8 nach Fig. 4 wird beispielsweise darauf hingewiesen, daß die Führungsbaugruppen 28, 31 und 33 in den angegebenen Schienensystemen durch einen Ritzel-Zahnstangenantrieb bewegbar sind. Hierbei wird jeweils das Ritzel von einer der Antriebsvorrichtungen angetrieben.

## Patentansprüche

1. Verfahren zur Mikroinjektion von Flüssigkeiten oder Suspensionen von Stoffen in lebende Zellen, mit einer bewegbar gelagerten Kanüle und einem Tisch, wobei die schräg zu diesem eingestellte Kanüle und der Tisch verbunden sind und diese Teile relativ zueinander in einer Ebene parallel zum Tisch in zwei Richtungen (x-y-Richtungen) einstellbar sind und ferner die Kanüle in einer zu der durch die x-y-Richtung bestimmten Ebene senkrechten z-Richtung bewegbar ist, dadurch gekennzeichnet, daß die Kanüle mit ihrer Spitze als Zeiger über einem gewünschten Injektionsort einer auf dem Tisch befindlichen Zelle in einer zum Tisch parallelen Ebene z₁ eingestellt wird, welche oberhalb des Zellenmaterials liegt, und daß die Kanüle in Abhängigkeit von dem Winkel der Kanule zu der genannten Ebene in einer Richtung, die durch die Kanülenrichtung in ihrer Projektion auf die Ebene bestimmt ist, um eine Strecke zurückbewegt wird, die so lang ist, daß die Kanüle am Ende der Rückwärtsbewegung in einer Stellung ist, in welcher die verlängerte Achsrichtung den gewünschten Injektionsort schneidet, und daß danach die Kanüle axial zum Einstich in die Zelle bewegt wird und diese Bewegung angehalten wird, wenn die Spitze den gewünschten Injektionsort erreicht hat.

2. Verfahren nach Anspruch 1, dadurch gekennzeichnet, daß oberhalb des Tisches eine zweite zu ihm parallele Ebene in Höhe z₀ über dem Tisch gewählt wird, welche Ebene im wesentlichen das Zellinnere der auf dem Tisch befindlichen Zellen schneidet, und daß die axiale Bewegung der Kanüle zum Einstich angehalten wird, wenn die Kanülenspitze die Ebene z₀ erreicht.

3. Verfahren nach Anspruch 1 und 2, dadurch gekennzeichnet, daß die Kanüle in einem Winkel von 45° zu der Ebene des Tisches eingestellt wird und die Rückwärtsbewegung gleich dem Abstand zwischen den Ebenen z₀ und z₁ ist.

4. Vorrichtung zur Durchführung des Verfahrens nach einem der Ansprüche 1 bis 3, mit einem Halter für die Kanüle und mit bewegbarem Kreuztisch, dadurch gekennzeichnet, daß der Halter (10) an einem Betätigungsgerät (8) einstellbar gelagert ist und eine Einstellungsanordnung zur Festlegung eines Winkels der Kanüle (11) zum Halter vorgesehen ist, daß dieses Betätigungsgerät wenigstens zwei Antriebsvorrichtungen (14, 16) aufweist, von denen eine erste Antriebsvorrichtung (14) zum Antrieb des Kanülen-Halters (10) in einer Richtung einer Ebene parallel zu einem zugeordneten Tisch und eine zweite Antriebsvorrichtung (16) zur Bewegung des Halters (10) in einer zu dieser Ebene senkrechten Richtung (z-Richtung) vorgesehen ist und daß Führungseinrichtungen zwischen dem Halter (10) und dem Betätigungsgerät (8) angeordnet sind, die in den Bewegungsrichtungen der wenigstens zwei Antriebsvorrichtungen verlaufen, und daß die wenigstens zwei Antriebsvorrichtungen zwischen diesem Halter (10) und einem Sockel des Betätigungsgerätes (8) vorgesehen sind, zu dem der Halter (10) in den Führungseinrichtungen bewegbar geführt ist.

5. Vorrichtung nach Anspruch 4, dadurch gekennzeichnet, daß zwei erste Antriebsvorrichtungen (14, 15) in der einen Ebene in x-y-Richtung vorgesehen sind.

6. Vorrichtung nach Anspruch 4 oder 5, dadurch gekennzeichnet, daß das Betätigungsgerät (8) mit einer Befestigungsanordnung (9) zur starren Verbindung mit einem Tisch ausgeführt ist.

7. Vorrichtung nach Anspruch 4 oder 5, dadurch gekennzeichnet, daß das Betätigungsgerät (8) einteilig mit einem Tisch ausgeführt ist.

8. Vorrichtung nach einem der Ansprüche 4 bis 7, dadurch gekennzeichnet, daß in Verbindung mit der zweiten Antriebsvorrichtung (16) eine Meßeinrichtung (24) vorgesehen ist, welche die einstellbare Höhendifferenz zwischen zwei Ebenen, und zwar einer z₀- und einer z₁-Ebene aufnimmt, welche Ebenen parallel zu der Ebene des Tisches verlaufen, und daß die Meßeinrichtung (24) ein entsprechendes Meßsignal in eine der ersten Antriebsvorrichtungen (14, 15) in der x-y-Richtung einspeist, welches Meßsignal unter Berücksichtigung der Neigung der Kanüle (11) zur z-Richtung in ihrer Anordnung im Halter (10) bemessen ist, wobei eine Steuereinrichtung (20) vorgesehen ist, welche eine erste Antriebsvorrichtung (14, 15) für eine Rückwärtsbewegung der Kanüle (11) in Abhängigkeit von dem Unterschied der Höhen zwischen der z₀- und z₁-Ebene bezüglich der Neigung der Kanüle (11) und ferner die zweite Antriebsvorrichtung (16) in z-Richtung gleichzeitig mit bemessener Antriebsbewegungsstrecke einschaltet.

9. Vorrichtung nach einem der Ansprüche 6 bis 8, dadurch gekennzeichnet, daß der Tisch ein Kreuztisch (4) mit Antriebsvorrichtungen (5, 6) in x-y-Richtung ist.

10. Vorrichtung nach Anspruch 9, dadurch gekennzeichnet, daß der Kreuztisch (4) Teil eines Mikroskopes ist.

## Claims

1. A process for microinjecting liquids or suspensions of substances into living cells by means of a movably mounted cannula and a stage, wherein the cannula extends at an oblique angle to the stage, the cannula and the stage are adjustable relative to each other in two directions (X, Y) in a plane which is parallel to the stage, and the cannula is also mobable in a direction (Z) which is at right angles to the plane that is defined by the directions (X and Y), characterized in that the pointer which is constituted by the tip of the cannula is moved to a position over a desired location for an injection into a selected cell disposed on the stage in a plane (z₁) that is parallel to the stage and disposed above the cells and that depending on the angle between the cannula to said plane in a direction of the vertical projection of said cannula on said parallel plane the cannula is subsequently moved back about a distance being so long that at the end of the setback movement the cannula is in a setback position in which the axis of said cannula extends through said desired location, and that an axial puncturing movement is imparted to said cannula from said setback position to puncture said selected cell, and said punturing movement is arrested when said tip of said cannula has reached said desired location.

2. The process according to claim 1, characterized in that a second plane parallel and above the stage is chosen in a height z₀ above the table which plane substantially intersects the cell interior of the cells on the stage, and that the cannulas axial movement for injection is stopped when the cannula tip reaches the plane z₀.

3. The process according to claims 1 and 2, characterized in that the cannula is adjusted in an angle of 45° to the plane of the table, and the setback movement is equal to the distance between the planes z₀ and z₁.

4. A device for carrying out the process according to any of the claims 1 to 3, with a holder for the cannula, and with a movable compassed stage, characterized in that the holder (10) is adjustably mounted at an actuation device (8), and an adjusting means for determining the angle of the cannula (11) to the holder is provided that this actuation device comprises at least two drive means (14, 16) from which a first drive means (14) is provided for driving the cannula holder (10) in a direction of a plane parallel to an adjoint stage, and a second drive means (16) is provided for the movement of the holder (10) in a direction (z-direction) perpendicular to that plane, and that guide means are arranged between the holder (10) and the actuation device (8) which pass along the direction of motion of said at least two drive means, and that said at least two drive means are provided between this holder (10) and a socket of the actuation device (8) to which the holder (10) is movably guided by the guide means.

5. The device according to claim 4, characterized in that two first drive means (14, 15) are provided in the one plane in x-y-direction.

6. The device according to claim 4 or 5, characterized in that the actuation device (8) is provided with a mounting means (9) for rigid connection to a stage.

7. The device according to claim 4 or 5, characterized in that the actuation device (8) is integral with a stage.

8. The device according to any of the claims 4 to 7, characterized in that in connection with the second drive means (16) a measuring device (24) is provided which takes up an adjustable height difference between two planes, specifically between a z₀ and a z₁ plane which planes are parallel to the plane of the stage, and that the measuring device (24) feeds a suitable measuring signal into one of the drive means (14, 15) in x-y-direction in which measuring signal is taken into account the inclination of the cannula (11) towards the z-direction in its arrangement in the holder (10), wherein a control means (20) is provided which activates a first drive means (14, 15) for the setback movement of the cannula (11) in dependence on the difference of the heights between the z₀ and the z₁ plane according to the inclination of the cannula (11), and furthermore activates the second drive means (16) in z-direction with rated driving motion path.

9. Device according to any of the claims 6 to 8, characterized in that the stgee is a cross positioning table (4) with driving devices (5, 6) in x-y-direction.

10. Device according to claim 9, characterized in that the cross positioning table (4) is part of a microscope.

## Revendications

1. Procédé pour micro-injection de liquides ou de suspensions de produits dans des cellules vivantes, comportant une canule montée mobile et une table, la canule, réglée inclinée par rapport à celle-ci, et la table étant reliées entre elles et ces pièces pouvant être réglées l'une par rapport à l'autre dans deux directions (directions x et y) dans un plan parallèle à la table, et, de plus, la canule pouvant être déplacée dans une direction Z perpendiculairement au plan défini par les directions x et y,
caractérisé en ce qu'on place la canule , avec sa pointe comme indicateur du point où on souhaite faire l'injection dans une cellule se trouvant sur la table, dans un plan Z₁ parallèle à la table et situé au dessus de la matière de la cellule,
en ce que, en fonction de l'angle de la canule par rapport audit plan dans une direction qui est déterminée par la direction de la projection de cette direction de canule, on recule celle-ci d'une longueur suffisante pour que la canule, à la fin du mouvement de recul, soit dans une position dans laquelle le prolongement de la direction de son axe rencontre le point d'injection souhaité, et
en ce qu'ensuite, la canule est déplacée axialement jusqu'à pénétrer dans la cellule et que ce mouvement est arrêté quand la pointe a atteint l'endroit d'injection souhaité.

2. Procédé suivant la revendication 1, caractérisé en ce qu'au-dessus de la table, on choisit un deuxième plan parallèle à la table, à une hauteur Z₀ au dessus d'elle, lequel plan recoupe essentiellement l'intérieur de la cellule se trouvant sur la table, et en ce que le mouvement axial de la canule est arrêté quand la pointe de la canule a atteint le plan Z₀.

3. Procédé suivant la revendication 1 et la revendication 2, caractérisé en ce que la canule est réglée dans un angle de 45° par rapport au plan de la table et en ce que le mouvement de recul est égal à la distance entre les plans Z₀ et Z₁.

4. Dispositif pour la mise en oeuvre du procédé suivant l'une quelconque des revendications 1 à 3, comportant un support de canule et une table mobile à mouvements croisés,
caractérisé en ce que le support (10) est monté réglable sur un appareil d'actionnement (8) et qu'il est prévu un système de réglage pour bloquer l'angle de la canule (11) par rapport au support, en ce que cet appareil d'actionnement présente au moins deux dispositifs d'entraînement (14, 16), dont le premier (14) est prévu pour entraîner le support de canule (10) dans la direction d'un plan parallèle à une table associée et dont le deuxième dispositif d'entraînement (16) est prévu pour faire déplacer le support (10) dans une direction perpendiculaire à ce plan (direction z),
en ce que des dispositifs de guidage sont placés entre le support (10) et l'appareil d'actionnement (8), et orientés dans les directions de déplacement des dispositifs d'entraînement, au moins au nombre de deux,
et en ce que ces dispositifs d'entraînement, au moins au nombre de deux, sont prévus entre ce support (10) et un socle de l'appareil d'actionnement (8), vers lequel est guidé le support (10), mobile dans les dispositifs de guidage.

5. Dispositif suivant la revendication 4, caractérisé en ce que deux premiers dispositifs d'entraînement (14, 15) sont prévus dans l'un des plans dans la direction x-y.

6. Dispositif suivant la revendication 4 ou la revendication 5, caractérisé en ce que l'appareil d'actionnement (8) est réalisé avec un système de fixation (9) ayant pour but de le relier de façon rigide avec une table.

7. Dispositif suivant la revendication 4 ou la revendication 5, caractérisé en ce que l'appareil d'actionnement (8) est réalisé en formant une seule pièce avec une table.

8. Dispositif suivant l'une quelconque des revendications 4 à 7, caractérisé en ce qu'il est prévu , en liaison avec le deuxième dispositif d'entraînement (16), un dispositif de mesure (24), qui relève la différence de hauteur, réglable, entre deux plans, en particulier un plan Z₀ et un plan Z₁ , ces plans étant parallèles au plan de la table,
et en ce que le dispositif de mesure (24) fait entrer un signal de mesure correspondant dans l'un des premiers dispositifs d'entraînement (14, 15) dans la direction x-y, lequel signal de mesure est mesuré en prenant en compte l'inclinaison de la canule (11) par rapport à la direction z dans sa disposition dans le support, étant entendu qu'il est prévu un dispositif de commande (20) qui enclenche un premier dispositif d'entraînement (14, 15) faisant reculer la canule (11) en fonction de la différence de hauteur entre les plans Z₀ et Z₁ par rapport à l'inclinaison de la canule (11), et enclenche , de plus, simultanément le deuxième dispositif d'entraînement (16) dans la direction (16), avec une course de déplacement mesurée.

9. Dispositif suivant l'une quelconque des revendications 6 à 8, caractérisé en ce que la table est une table à mouvements croisés (4), comportant des dispositifs d'entraînement (5, 6) dans la direction x-y.

10. Dispositif suivant la revendication 9, caractérisé en ce que la table à mouvements croisés (4) est une partie d'un microscope.
